(19) Europäisches Patentamt / European Patent Office / Office européen des brevets

(11) **EP 3 712 855 B1**

(12) **EUROPÄISCHE PATENTSCHRIFT**

(45) Veröffentlichungstag und Bekanntmachung des Hinweises auf die Patenterteilung:
**07.01.2026 Patentblatt 2026/02**

(21) Anmeldenummer: **19164362.6**

(22) Anmeldetag: **21.03.2019**

(51) Internationale Patentklassifikation (IPC):
**G06T 15/08** (2011.01)     **G06T 19/20** (2011.01)
**G06T 19/00** (2011.01)

(52) Gemeinsame Patentklassifikation (CPC):
**G06T 19/20; G06T 15/08; G06T 19/00;**
G06T 2210/41; G06T 2219/016

(54) **VISUALISIERUNG VON MEDIZINISCHEN BILDDATEN**

VISUALIZATION OF MEDICAL IMAGE DATA

VISUALISATION DE DONNÉES D'IMAGES MÉDICALES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.09.2020 Patentblatt 2020/39**

(73) Patentinhaber: **Siemens Healthineers AG**
**91301 Forchheim (DE)**

(72) Erfinder:
• **Engel, Klaus**
**90491 Nürnberg (DE)**
• **Hufnagel, Simone**
**90559 Burgthann (DE)**

(74) Vertreter: **Siemens Healthineers**
**Patent Attorneys**
**Postfach 22 16 34**
**80506 München (DE)**

(56) Entgegenhaltungen:
US-A- 5 825 365

• **STEFAN BRUCKNER ET AL: "Exploded Views for Volume Data", IEEE TRANSACTIONS ON VISUALIZATION AND COMPUTER GRAPHICS, IEEE SERVICE CENTER, LOS ALAMITOS, CA, US, vol. 12, no. 5, 1 September 2006 (2006-09-01), pages 1077 - 1084, XP011150904, ISSN: 1077-2626, DOI: 10.1109/TVCG.2006.140**

• **KANG D S ET AL: "An interactive exploded view generation using block-based re-rendering", MEDICAL IMAGING 2011: VISUALIZATION, IMAGE-GUIDED PROCEDURES, AND MODELING, SPIE, 1000 20TH ST. BELLINGHAM WA 98225-6705 USA, vol. 7964, no. 1, 3 March 2011 (2011-03-03), pages 1 - 7, XP060008164, [retrieved on 20110301], DOI: 10.1117/12.877895**

• **SUBRAMANIAN N ET AL: "Volume rendering segmented data using 3D textures: a practical approach for intra-operative visualization", PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA, vol. 6141, 2006, pages 61412Q - 1, XP002793564, ISSN: 0277-786X**

• **HENRY SONNET ET AL: "Integrating expanding annotations with a 3D explosion probe", AVI'04 INTERNATIONAL CONFERENCE ON ADVANCED VISUAL INTERFACES; GALLIPOLI, ITALY; MAY 25 - 28, 2004, ASSOCIATION FOR COMPUTING MACHINERY, NEW YORK, NY, USA, 25 May 2004 (2004-05-25), pages 63 - 70, XP058274606, ISBN: 978-1-58113-867-2, DOI: 10.1145/989863.989871**

• **WILMOT LI ET AL: "Automated generation of interactive 3D exploded view diagrams", ACM TRANSACTIONS ON GRAPHICS, ACM, 2 PENN PLAZA, SUITE 701NEW YORKNY10121-0701USA, vol. 27, no. 3, 1 August 2008 (2008-08-01), pages 1 - 7, XP058355394, ISSN: 0730-0301, DOI: 10.1145/1360612.1360700**

EP 3 712 855 B1

**EP 3 712 855 B1**

**Beschreibung**

Die Erfindung betrifft ein Verfahren zur Visualisierung von medizinischen Bilddaten als Volumendaten. Bei dem Verfahren werden medizinische Bilddaten akquiriert. Zudem wird eine dreidimensionale Maske erzeugt. Zur Erzeugung der dreidimensionalen Maske werden die Bilddaten segmentiert und die segmentierten Bereiche werden in vorbestimmte Klassen eingeteilt. Weiterhin betrifft die Erfindung eine Visualisierungseinrichtung. Die Visualisierungseinrichtung umfasst eine Akquisitionseinheit zum Akquirieren von medizinischen Bilddaten. Weiterhin weist die Visualisierungseinrichtung eine Maskenerzeugungseinheit zum Erzeugen einer 3D-Maske durch Segmentieren der Bilddaten und Einteilen der segmentierten Bereiche in vorbestimmte Klassen auf. Überdies betrifft die Erfindung ein medizintechnisches Bildgebungssystem. Das medizintechnische Bildgebungssystem umfasst eine Bildaufnahmeeinheit zum Erzeugen von Bilddaten von einem Untersuchungsbereich eines zu untersuchenden Objekts.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können. Die dabei gewonnenen Bilddaten, wie sie bei der Computertomographie oder der Magnetresonanzbildgebung erzeugt werden, werden meistens als Schichtbilder zur Begutachtung durch medizinisches Fachpersonal zur Verfügung gestellt.

Allerdings sind zur besseren Veranschaulichung auch dreidimensionale Bilddarstellungen, auch Volumengrafiken genannt, erforderlich. Die für solche Darstellungen benötigten volumetrischen Daten werden durch ein sogenanntes Volume-Rendering dreidimensional visualisiert, um eine fallspezifische Anatomie zu veranschaulichen. Dabei bildet ein bildlich erfasster Volumenbereich zunächst eine Einheit und kann daher nicht ohne Weiteres in einzelne anatomische Strukturen zerlegt werden, um diese zum Beispiel genauer zu begutachten.

Die Volumenbilddarstellung basiert auf der Technik des sogenannten Renderns. Zunächst liegen Bilddaten als Schichtbilddaten vor, wobei jedem Voxel, zumindest im Fall der Computertomographie, ein Grauwert entsprechend der ermittelten Dichte an dieser Stelle zugeordnet ist. Diese Daten lassen sich jedoch bei einer dreidimensionalen perspektivischen Visualisierung nicht direkt in Bilddaten umsetzen, da für eine solche Visualisierung die Beobachtungsperspektive und die Position der einzelnen Voxel relativ zum Beobachter mitberücksichtigt werden muss. Außerdem weisen einzelne Voxel zunächst nur jeweils einen einzigen Wert auf, welcher bei der CT-Bildgebung beispielsweise Auskunft über die Röntgendichte oder bei der MR-Bildgebung Auskunft über den Gehalt an Protonen oder Wasserstoffatomkernen gibt, aber sie enthalten keine Informationen über das Aussehen des jeweiligen Materials eines Voxels, d.h. zum Beispiel über dessen Farbe oder darüber, ob es stark spiegelnd ist. Es wird daher jedem Voxel eines betrachteten Volumens eine Textur zugewiesen. Dieser Vorgang wird auch als Klassifikation bezeichnet. Ein weiterer Schritt befasst sich mit der Schattierung, d.h. wieviel Licht von einem Voxel aus in Richtung des Betrachters reflektiert wird und welche Farbe es hat. Weiterhin erfolgt ein Abtastschritt zum Abtasten des Volumens. Dabei werden Sehstrahlen in das abzubildende Volumen hineingeworfen. Nachdem die Interaktion des Lichts mit dem Volumen berechnet ist, werden die Beiträge entlang des Sehstrahls aufsummiert und ergeben so ein Pixel im Bild. Ein solches Vorgehen wird zum Beispiel durch das sogenannte Raycasting realisiert. Es ist jedoch herkömmlich nicht möglich nur einen Teil der erzeugten Volumendaten darzustellen.

Oft ist jedoch eine solche detaillierte Visualisierung von Teilbereichen des gesamten Aufnahmebereichs wünschenswert. Zur Darstellung von anatomischen Details werden herkömmlich sogenannte polygonale Oberflächenmodelle genutzt. Bei diesen dient ein künstlich erstelltes Modell als Datengrundlage für die Visualisierung. Dadurch kann jede Struktur einzeln modelliert und unabhängig voneinander im Bild bewegt werden. Allerdings lässt sich bei einer Oberflächendarstellung das Innere von anatomischen Strukturen nicht untersuchen. Beispielsweise ist es nicht möglich, ein Organ virtuell aufzuschneiden und nach Pathologien zu untersuchen, die von außen eventuell nicht sichtbar sind. Außerdem sind zur Darstellung mit Hilfe von Oberflächenmodellen sogenannte Zwischenrepräsentationen notwendig, welche einen hohen Datenaufwand erfordern und zu fehlerhaften Darstellungen beitragen.

In Stefan Bruckner et al: "Exploded Views for Volume Data", IEEE TRANSACTIONS ON VISUALIZATION AND COMPUTER GRAPHICS, IEEE SERVICE CENTER, LOS ALAMITOS, CA, US, Bd. 12, Nr. 5, 1. September 2006 (2006-09-01), Seiten 1077-1084, XP011150904, ISSN: 1077-2626, DOI: 10.1109/TVCG.2006 wird die Darstellung von Explosionsansichten von Volumendaten beschrieben. Zur Realisierung der Explosionsansichten werden auch sogenannte Raycasting-Verfahren eingesetzt.

In Kang D S et al: "An interactive exploded view generation using block-based re-rendering", MEDICAL IMAGING 2011: VISUALIZATION, IMAGE-GUIDED PROCEDURES, AND MODELING; SPIE, 1000 20TH ST. BELLINGHAM WA 98225-6705 USA, BD. 7964, Nr. 1, 3. März 2011 (2011-03-03), Seiten 1-7, XP060008164, DOI: 10.1117/12.877895 wird die Erzeugung von Explosionsdarstellungen unter Anwendung eines block-basierten Re-rendering-Verfahrens beschrieben.

In Subramanian N et al: "Volume rendering segmented data using 3D textures: a practical approach for intraoperative visualization", PROCEEDINGS OF THE SPIE - THE INTERNATIONAL SOCIETY FOR OPTICAL ENINEERING USA, Bd. 6141, 2006, Seiten 614120-1, XP002793564, ISSN: 0277-786X wird ein Volumen-Rendering-Verfahren auf Basis von 3D-Texturen beschrieben.

In HENRY SONNET ET AL: "Integrating expanding annotations with a 3D explosion probe", AVI'04 INTERNATIONAL CONFERENCE ON ADVANCED VISUAL INTERFACES; GALLIPOLI, ITALY; MAY 25-28, 2004, ASSOCIATION FOR COMPUTING MACHINERY, NEW YORK, NY, USA, 25. Mai 2004 (2004-05-25), Seiten 63-70, XP058274606, DOI:10.1145/989863.989871 ISBN: 978-1-58113-867-2 wird eine um Annotationen angereichte Explosionsdarstellung beschrieben.

In US 5,825,365 wird ein Verfahren zur dreidimensionalen dynamischen Darstellung beschrieben.

In WILMOT Li et al: "Automated generation of interactive 3D exploded view diagrams", ACM TRANSACTIONS ON GRAPHCS, ACM, 2 PENN PLAZA, SUITE 701 NEW YORKNY10121-0701 USA, Bd. 27, Nr. 3, 1. August 2008 (2008-08-01), Seiten 1-7, XP058355394, ISSN:0730-0301, DOI:10.1145/1360612.1360700 wird eine automatisierte Erzeugung interaktiver Explosionsdarstellungen beschrieben.

Es ist mithin Aufgabe der vorliegenden Erfindung, eine genauere und vereinfachte Darstellung auch von einzelnen anatomischen Strukturen auf Basis dreidimensionaler Bilddaten von einem Untersuchungsbereich eines Untersuchungsobjekts zu ermöglichen.

Diese Aufgabe wird durch ein Verfahren zur Visualisierung von medizinischen Bilddaten als Volumendaten, eine Visualisierungseinrichtung, ein medizintechnisches Bildgebungssystem, ein Computerprogrammprodukt sowie einen Computerlesbaren Datenträger gemäß den unabhängigen Ansprüchen gelöst.

Bei dem erfindungsgemäßen Verfahren zur Visualisierung von medizinischen Bilddaten als Volumendaten werden zunächst medizinische Bilddaten von einem zu untersuchenden Objekt akquiriert. Die Bilddaten können zum Beispiel von einer medizintechnischen bildgebenden Einrichtung, wie zum Beispiel ein CT-System oder ein MR-System, kommen. Die Bilddaten werden anschließend vorzugsweise automatisiert segmentiert und die segmentierten Bereiche werden in vorbestimmte, unterschiedliche Organe bzw. Strukturarten kennzeichnende Klassen eingeteilt. Auf diese Weise wird eine Maske erzeugt, die einzelnen segmentierten Volumenelementen jeweils Marken bzw. Label zuweist, um sie zu charakterisieren. Mit Hilfe dieser Marken kann ein Voxel eindeutig einer bestimmten Organart oder Gewebeart zugeordnet werden. Die Bilddaten und die Maskendaten werden in zwei getrennten 3D-Texturen-Dateien abgespeichert.

In einer dieser Texturen-Dateien befinden sich die Informationen über das Aussehen der einzelnen Voxel und in der anderen Datei befinden sich die aus der Segmentierung bzw. Maskierung gewonnenen Informationen darüber, welcher Struktur oder welchem Organ ein jeweiliges Voxel zuzurechnen ist. Die Datei für die "Bilddaten" umfasst nicht nur die seitens der medizintechnischen bildgebenden Einrichtungen erzeugten Dichte- oder Konzentrationsinformationen, sondern auch Volumentexturdaten, welche das Aussehen eines Voxels, wie bereits kurz erläutert, genauer definieren. Weiterhin wird ein Translationsvektor ermittelt, welcher die Verschiebung eines segmentierten Volumenelements zwischen einer Ursprungsposition und einer Zielposition beschreibt. Die Ursprungsposition und die Zielposition können von einem Benutzer zum Beispiel mit Hilfe eines Eingabemediums vorgegeben werden. Außerdem wird eine bildliche Darstellung der Bilddaten durch Anwenden eines Strahlabtastverfahrens auf die abgespeicherten Bilddaten erzeugt. Weiterhin erfolgt eine Verschiebung eines segmentierten Volumenelements in der bildlichen Darstellung um den Translationsvektor. D.h., das segmentierte Volumenelement wird in der bildlichen Darstellung um den Translationsvektor verschoben. Die Verschiebung kann zum Beispiel durch eine durch den Benutzer gesteuerte Bewegung des Eingabemediums ausgelöst werden. Die Verschiebung erfolgt dabei derart, dass die Bildpunkte im Zielbereich durch eine um den Translationsvektor verschobene Abtastung erzeugt werden, so dass die Bildpunkte im Zielbereich die Texturdaten des Ursprungsbereichs erhalten. Die Verschiebung von Volumendaten ermöglicht eine verbesserte Veranschaulichung von Teilbereichen des zu untersuchenden Objekts.

Die erfindungsgemäße Visualisierung von volumetrischen Bilddaten ermöglicht im Gegensatz zu der Anwendung von polygonalen Oberflächenmodellen auch eine Veranschaulichung des Innenlebens von anatomischen Strukturen. So kann beispielsweise ein Organ virtuell aufgeschnitten und nach pathologischen Phänomenen untersucht werden, die äußerlich nicht sichtbar sind. Außerdem werden bei der Visualisierung von Volumendaten keine Zwischenrepräsentationen benötigt, wodurch der Datenaufwand reduziert und Artefakte bei der Visualisierung vermieden werden können.

Außerdem wird der Datenaufwand auch dadurch reduziert, dass trotz einer Translation von Segmenten nur ein und derselbe Datensatz im Datenspeicher gehalten werden muss. Anstatt Texturen im Datensatz zu verändern und dadurch erhöhten Speicherplatz zu benötigen, erfolgt die Manipulation der Bilddaten erst bei der Abtastung zur Visualisierung der Bilddaten. Der sogenannte Rendering-Vorgang kann also unabhängig davon, ob und welche Verschiebung vorgenommen wird, immer auf Basis derselben Daten und derselben Routine ablaufen. Überdies werden für die Verschiebung nur drei unterschiedliche Eingaben benötigt: die Bilddaten mit einer Volumentextur, eine Maske mit Labels für die Segmentierung und ein Translationsvektor für die Verschiebung eines Segments.

Die erfindungsgemäße Visualisierungseinrichtung weist eine Akquisitionseinheit zum Akquirieren der medizinischen Bilddaten auf. Teil der erfindungsgemäßen Visualisierungseinrichtung ist auch eine Maskenerzeugungseinheit zum Erzeugen einer 3D-Maske durch Segmentieren der Bilddaten und Einteilen der segmentierten Bereiche in vorbestimmte Klassen. Die erfindungsgemäße Visualisierungseinrichtung umfasst auch eine Speichereinheit zum Abspeichern der Bilddaten und der Maskendaten in zwei getrennten 3D-Textur-Dateien.

Die erfindungsgemäße Visualisierungseinrichtung weist zudem eine Vektorerzeugungseinheit zum Berechnen eines Translationsvektors, welcher die Verschiebung eines segmentierten Volumenelements zwischen einer Ursprungsposition und einer Zielposition beschreibt, auf. Die Verschiebung bzw. der Translationsvektor kann zum Beispiel durch eine durch den Benutzer gesteuerte Bewegung eines Eingabemediums oder durch eine von dem Benutzer angesteuerte Ursprungs- und Zielposition eines Eingabemediums festgelegt werden.

Überdies weist die erfindungsgemäße Visualisierungseinrichtung auch eine Translationseinheit zum Verschieben des segmentierten Volumenelements um den Translationsvektor auf. Teil der erfindungsgemäßen Visualisierungseinrichtung ist auch eine Visualisierungseinheit zum Erzeugen einer bildlichen Darstellung der Bilddaten durch Anwendung eines Strahlabtastverfahrens auf die abgespeicherten Bilddaten. Die erfindungsgemäße Visualisierungseinrichtung teilt die Vorteile des erfindungsgemäßen Verfahrens zur Visualisierung von medizinischen Bilddaten als Volumendaten.

Das erfindungsgemäße medizintechnische Bildgebungssystem weist die erfindungsgemäße Visualisierungseinrichtung und eine Bildaufnahmeeinheit zum Erzeugen von Bilddaten von einem Untersuchungsbereich eines zu untersuchenden Objekts auf.

Die Bildaufnahmeeinheit kann zum Beispiel eine CT-Scaneinheit oder eine MR-Scaneinheit oder eine Tomosyntheseeinheit umfassen.

Das erfindungsgemäße medizintechnische Bildgebungssystem teilt die Vorteile der erfindungsgemäßen Visualisierungseinrichtung.

Teile der erfindungsgemäßen Visualisierungseinrichtung können zum überwiegenden Teil in Form von Softwarekomponenten ausgebildet sein. Dies betrifft insbesondere Teile der Akquisitionseinheit, der Maskenerzeugungseinheit, der Vektorerzeugungseinheit, der Translationseinheit und der Visualisierungseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine teilweise softwaremäßige Realisierung hat den Vorteil, dass auch schon bisher in bildgebenden Systemen genutzte Rechnersysteme bei einer Ergänzung um Hardwareeinheiten zur Aufnahme von Bilddaten von dem Patienten, auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines solchen Rechnersystems ladbar ist, mit Programmabschnitten, um alle Schritte des Verfahrens zur Visualisierung von medizinischen Bilddaten als Volumendaten auszuführen, wenn das Computerprogramm in dem Rechnersystem ausgeführt wird.

Ein solches Computerprogrammprodukt kann neben dem Computerprogramm gegebenenfalls zusätzliche Bestandteile, wie z.B. eine Dokumentation und/oder zusätzliche Komponenten, auch Hardware-Komponenten, wie z.B. Hardware-Schlüssel (Dongles etc.) zur Nutzung der Software, umfassen

Zum Transport zur Speichereinrichtung des Rechnersystems und/oder zur Speicherung an dem Rechnersystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche zu neuen Ausführungsbeispielen kombiniert werden.

Bei einer besonders bevorzugten Weiterbildung des erfindungsgemäßen Verfahrens zur Visualisierung von medizinischen Bilddaten als Volumendaten wird das segmentierte Volumenelement an der ursprünglichen Position transparent gemacht. Ein transparentes Verhalten kann durch eine Nullsetzung eines Werts der Opazität für das segmentierte Volumenelement an der ursprünglichen Position erreicht werden. Vorteilhaft tritt das zu verschiebende Volumenelement nicht doppelt in der bildlichen Darstellung auf. Auf diese Weise wird eine Verdeckung von anderen Strukturen an der ursprünglichen Position in der bildlichen Darstellung vermieden.

In einer Ausgestaltung des erfindungsgemäßen Verfahrens zur Visualisierung von medizinischen Bilddaten als Volumendaten wird ein Collider-Objekt für jedes segmentierte Volumenelement erstellt, dessen Form an die Maße des segmentierten Volumenelements angepasst wird. Ein solches Collider-Objekt ist eine Struktur, die einem dreidimensionalen Objekt angehängt werden kann. Das Collider-Objekt selbst nimmt die Form des dreidimensionalen Objekts an und ist dafür zuständig, ein Ereignis einer Kollision bzw. Berührung des dreidimensionalen Objekts mit anderen Collider-Objekten zu registrieren und an andere Schnittstelle weiterzugeben. Weiterhin wird der Translationsvektor aus der Differenz der aktuellen Position eines Eingabemediums und der Position, an der das Eingabemedium zum ersten Mal das Collider-Objekt berührt und gleichzeitig der Nutzer angibt, das segmentierte Volumenelement verschieben zu wollen, ermittelt. Mit dieser Variante kann ein Verschiebungseffekt für einzelne hervorzuhebende Strukturen eines zu untersuchenden Bereichs erzielt werden.

In einer speziellen Ausgestaltung des erfindungsgemäßen Verfahrens zur Visualisierung von medizinischen Bilddaten als Volumendaten wird ein Abstand zwischen dem zu verschiebenden segmentierten Volumenelement und dem übrigen Volumen vorbestimmt und es ergibt sich der Translationsvektor aus dem Produkt einer Differenz aus einem Abstandsfaktor und dem Wert 1 und einer Differenz aus einer Position des Zentrums des zu verschiebenden segmentierten Volumenelements an seiner Ausgangsposition und einer aktuellen Position eines Eingabemediums. Dabei hängt die Länge des Translationswegs sowohl von einem vorbestimmten Abstandsfaktor als auch von dem Abstand zwischen dem Eingabemedium und dem zu verschiebenden Segment ab. Einzelne anatomische Strukturen können auf diese Weise herausgehoben werden und besser sichtbar gemacht werden. Vorteilhaft kann eine Art Explosionsdarstellung einzelner oder mehrerer unterschiedlicher anatomischer Strukturen erzielt werden und in dieser Darstellung können die anatomischen Strukturen dann näher untersucht werden.

In einer Variante des erfindungsgemäßen Verfahrens zur Visualisierung von medizinischen Bilddaten als Volumendaten werden nahe an dem Eingabemedium liegende segmentierte Volumenelemente stärker verschoben als von dem Eingabemedium entfernter liegende segmentierte Volumenelemente.

Die Erfindung wird im Folgenden unter Hinweis auf die beigefügten Figuren anhand von Ausführungsbeispielen noch einmal näher erläutert. Es zeigen:

FIG 1    ein Flussdiagramm, welches ein Verfahren zur Visualisierung von medizinischen Bilddaten als Volumendaten gemäß einem Ausführungsbeispiel der Erfindung veranschaulicht,

FIG 2    eine schematische Darstellung einer Verschiebung eines Segments von visualisierten Volumendaten,

FIG 3    ein Flussdiagramm, welches ein Verfahren zur Visualisierung von medizinischen Bilddaten als Volumendaten gemäß einem alternativen Ausführungsbeispiel der Erfindung veranschaulicht,

FIG 4    eine schematische Darstellung einer Verschiebung eines Segments von visualisierten Volumendaten in dem alternativen Ausführungsbeispiel,

FIG 5    eine schematische Darstellung einer Visualisierungseinrichtung gemäß einem Ausführungsbeispiel der Erfindung,

FIG 6    eine schematische Darstellung eines medizintechnischen Bildgebungssystems gemäß einem Ausführungsbeispiel der Erfindung.

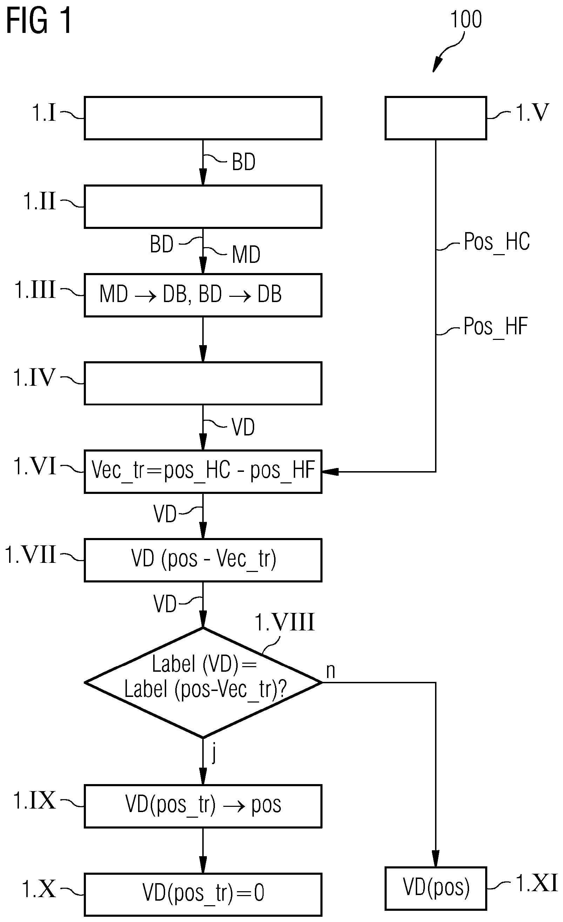

In FIG 1 ist ein Flussdiagramm 100 gezeigt, welches ein Verfahren zur Visualisierung von medizinischen Bilddaten als Volumendaten gemäß einem Ausführungsbeispiel der Erfindung veranschaulicht. Bei dem Schritt 1.I werden zunächst Bilddaten BD eines zu untersuchenden Bereichs eines Patienten von einer medizintechnischen Bildgebungseinrichtung empfangen. Diese Bilddaten BD liegen in Form von Grauwerten vor, die ortsabhängig variieren. Die Bilddaten BD werden bei dem Schritt 1.II segmentiert. D.h., es werden einzelne Organe oder andere Körperstrukturen identifiziert und voneinander abgegrenzt. Dieser Vorgang kann vollautomatisiert, teilautomatisiert oder auch durch qualifiziertes Personal erfolgen, welches in den Bilddaten BD Grenzlinien einzeichnet, um auf diese Weise die Bilddaten in segmentierte Bereiche einzuteilen. Bei der Segmentierung wird eine 3D-Maske MD erstellt und Bereichen im Bildvolumen werden bestimmte Klassen bzw. Gewebearten zugewiesen. Diese Maske ordnet einzelnen Voxeln sogenannte Label zu, die ein Voxel einem Segment bzw. einem Segmenttyp eindeutig zuordnen. Auf diese Weise bekommt man zwei Datensätze, einen Volumendatensatz mit den Grauwerten der Bilddaten BD und einen Maskendatensatz MD mit den Maskendaten, welche jedes Voxel einem bestimmten Segment bzw. Körperteil zuordnen. Weiterhin werden den Bilddaten BD Texturinformationen zugeordnet, welche sich aus der Kenntnis der den einzelnen Organen oder Gewebearten zugeordneten optischen Merkmale, wie zum Beispiel deren Farbe oder Spiegeleffekte ergeben.

Diese beiden Datensätze BD, MD werden bei dem Schritt 1.III als zwei getrennte Texturen bzw. in zwei getrennten Texturdateien abgespeichert. Anschließend erfolgt bei dem Schritt 1.IV eine Visualisierung der Bilddaten als Volumendaten VD durch ein Strahlabtastverfahren (auf englisch Raycasting). Bei diesem Strahlabtastverfahren werden virtuelle Strahlen in das Bildvolumen gelegt und es wird auf Basis der abgetasteten Bilddaten BD ein Bild von einem zu untersuchenden Bereich erzeugt, das aus entsprechenden visualisierten Volumendaten VD aufgebaut ist. In dem visualisierten Bild kann nun ein Benutzer einzelne Segmente anwählen und verschieben.

Hierzu wird bei dem Schritt 1.V von einem Benutzer mit Hilfe eines Eingabemittels, beispielsweise einer Hand oder einem sogenannten Controller, ein zu verschiebendes Segment angewählt. Der Benutzer deutet zum Beispiel mit Hilfe des Eingabemediums auf einer Bildanzeige auf eine zu verschiebende Struktur an einer Position pos_HF, an der sich diese zu verschiebende Struktur im Bild befindet und gibt an, diese Struktur verschieben zu wollen. Auf Basis der bekannten Position des Zentrums der zu verschiebenden Struktur und deren Ausmaße im Bild wird nun ein sogenanntes Collider-Objekt der Struktur erstellt, dessen Form an die Maße der zu verschiebenden Struktur angepasst ist. Hierfür werden für das angewählte Segment dessen Zentrum und auch dessen Ausmaße bzw. Grenzen ermittelt. Dieser Vorgang kann anhand der abgespeicherten Maskendaten MD erfolgen, welche jeden Bildpunkt einem Segment zuordnen. Weiterhin gibt der Benutzer durch eine Bewegung des Eingabemediums an die Zielposition pos_HC an, an welche Stelle im visualisierten Bildvolumen die Struktur verschoben werden soll.

Bei dem Schritt 1.VI wird ein Translationsvektor Vec_tr auf Basis einer Verschiebungsbewegung eines Benutzers ermittelt. Genauer gesagt, wird auf Basis der Ausgangsposition pos_HF und der Zielposition pos_HC der Translationsvektor Vec_tr zu

$$Vec\_tr = pos\_HC - pos\_HF \qquad\qquad (1)$$

berechnet.

Nachdem der Translationsvektor Vec_tr ermittelt wurde, wird noch der Zugriff auf die Texturkoordinaten angepasst. Denn ansonsten würde der Dichtewert bzw. eine Textur eines Abtastvolumens an einer aktuellen Abtastposition pos durch Auslesen der Bilddaten BD und deren zugeordnete Textur an der aktuellen Position pos visualisiert werden. Aufgrund der Translation um den Translationsvektor Vec_tr wird aber bei dem Schritt 1.VII eine um den Translationsvektor Vec_tr verschobene Position pos_tr = pos - Vec_tr abgetastet.

Falls die Maskendaten MD an dieser Position das Label der zu verschiebenden Struktur aufweisen, was bei dem Schritt 1.VIII geprüft wird, bzw. in FIG 1 mit "j" gekennzeichnet ist, wird nun bei dem Schritt 1.IX der Abtastposition pos der Dichtewert bzw. die Texturdaten der visualisierten Volumendaten VD (pos_tr) und nicht der Volumendaten VD (pos) zugwiesen, so dass die aktuelle Texturkoordinate an der Stelle pos im visualisierten Bild verändert wird. Zusätzlich erfolgt bei dem Schritt 1.X eine Änderung der Textur an der verschobenen Position pos_tr, derart, dass die zu verschiebende Struktur an der ursprünglichen Position pos_tr transparent ist. Auf diese Weise wird eine doppelte Darstellung der zu verschiebenden Struktur vermieden.

Falls der verschobenen Position pos_tr kein Label der zu verschiebenden Struktur zugeordnet ist, was in FIG 1 mit "n" gekennzeichnet ist, wird bei dem Schritt 1.XI der Abtastposition pos der Dichtewert der ursprünglichen Volumendaten VD (pos) zugewiesen und es erfolgt somit keine Veränderung des visualisierten Bildes an dieser Stelle. D.h. in diesem Fall befindet sich die aktuelle Abtastposition pos außerhalb des Bereichs der zu verschiebenden Struktur.

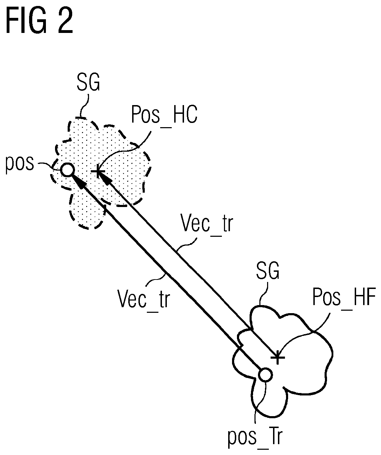

In FIG 2 ist eine schematische Darstellung einer Verschiebung eines Segments SG von visualisierten Volumendaten veranschaulicht. Das Segment SG soll um den Translationsvektor Vec_tr von der Position pos_HF an die aktuelle Position pos_HC verschoben werden. Einem Bildpunkt an der Stelle pos wird nun nicht die Textur der Position pos in den Volumendaten zugeordnet, sondern die Textur der um den Translationsvektor Vec_tr verschobenen Position pos_Tr. Auf diese Weise wird die zu verschiebende Struktur SG von der Position pos_Tr an die Stelle pos verschoben.

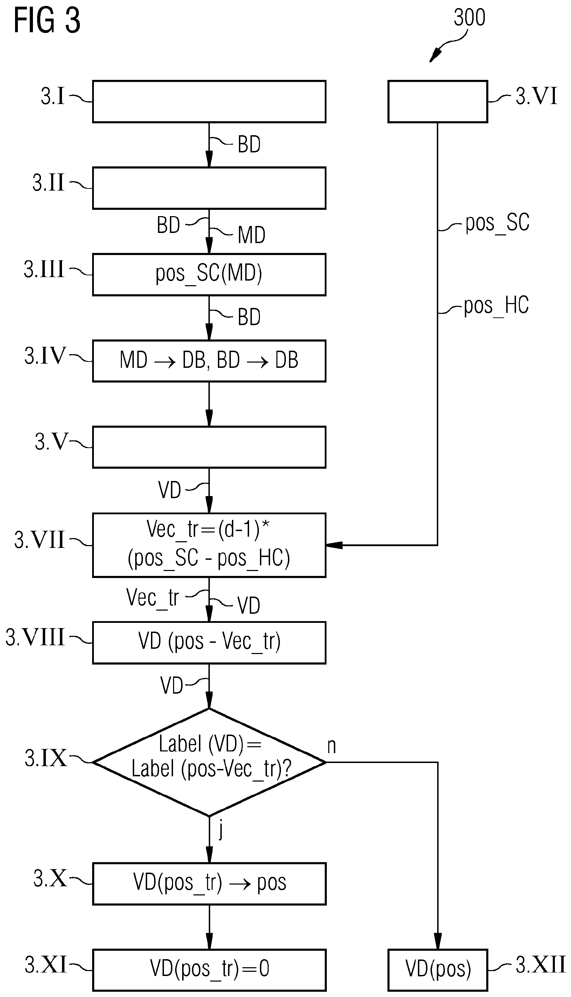

In FIG 3 ist ein Flussdiagramm gezeigt, welches ein Verfahren zur Visualisierung von medizinischen Bilddaten als Volumendaten gemäß einem alternativen Ausführungsbeispiel der Erfindung veranschaulicht. Das in FIG 3 veranschaulichte Ausführungsbeispiel unterscheidet sich im Wesentlichen von dem in FIG 1 veranschaulichten Ausführungsbeispiel dadurch, dass der Translationsvektor Vec_tr anders definiert ist. Auf diese Weise wird durch ein Verschieben einer ausgewählten Struktur der Abstand zwischen den Strukturen im visualisierten Bildvolumen in einem definierten Bereich um die Position des Eingabemediums herum ähnlich wie bei einer Explosionszeichnung vergrößert. Eine solche Darstellung ermöglicht es, einzelne Strukturen genauer begutachten zu können.

Bei dem Schritt 3.I werden zunächst wie bei dem in FIG 1 veranschaulichten Ausführungsbeispiel Bilddaten BD eines zu untersuchenden Bereichs eines Patienten von einer medizintechnischen Bildgebungseinrichtung empfangen. Diese Bilddaten BD liegen in Form von Grauwerten vor, die ortsabhängig variieren. Die Bilddaten BD werden bei dem Schritt 3.II segmentiert. Bei der Segmentierung wird eine 3D-Maske MD erstellt und es werden segmentierte Bereiche im Bildvolumen unterschiedlichen Klassen bzw. Gewebearten zugewiesen. Die Maske MD weist einzelnen Voxeln sogenannte Label zu, die ein Voxel einem Segment eindeutig zuordnen. Auf diese Weise bekommt man zwei Datensätze, einen Bilddatendatensatz BD mit den Grauwerten der Bilddaten und Texturinformationen und einen Maskendatensatz MD mit den Maskendaten, welche jedes Voxel einem bestimmten Segment bzw. Körperteil zuordnen.

Bei dem Schritt 3.III wird für jedes der Segmente bzw. Strukturen die Position pos_SC des Zentrums der jeweiligen Struktur ermittelt. Ein solches Zentrum kann zum Beispiel als Koordinatenschwerpunkt eines Segments ermittelt werden.

Der Bilddatensatz BD sowie der Maskendatensatz MD werden dann bei dem Schritt 3.IV als zwei getrennte Texturen abgespeichert.

Anschließend erfolgt bei dem Schritt 3.V eine Visualisierung von Volumendaten VD durch ein Strahlabtastverfahren (auf englisch Raycasting), welches bereits im Zusammenhang mit FIG 1 kurz erläutert wurde. In dem visualisierten Bild kann nun ein Benutzer einzelne Segmente anwählen und verschieben.

Bei dem Schritt 3.VI erfolgt durch den Benutzer mit Hilfe eines Eingabemediums eine Auswahl eines exponiert darzustellenden Segments in der visualisierten Darstellung. Beispielsweise nähert sich der Benutzer mit dem Eingabemedium aus einer bestimmten Richtung dem zu exponierenden Segment bis auf eine aktuelle Position pos_HC.

Bei dem Schritt 3.VII wird ein Translationsvektor Vec_tr ermittelt. Bei dem in FIG 3 veranschaulichten Ausführungsbeispiel wird nun die von dem Benutzer ausgewählte Struktur von ihrem Zentrum pos_SC um eine durch einen vorbestimmten Distanzfaktor dist bestimmte Distanz in Richtung der aktuellen Position pos_HC des Eingabemediums verschoben. Der dimensionslose Distanzfaktor dist kann vorab beliebig gewählt werden. Beispielsweise können einzelne Strukturen alle um denselben Faktor verschoben werden oder nah an dem Eingabemedium liegende Strukturen können stärker verschoben werden als weiter entfernte Strukturen.

Auf Basis der Ausgangsposition bzw. der Zentrumsposition pos_SC, der aktuellen Position des Eingabemediums pos_HC und des Distanzfaktors dist wird nun der Translationsvektor Vec_tr zu

$$\text{Vec\_tr} = (\text{dist} - 1) * (\text{pos\_SC} - \text{pos\_HC}) \quad (2)$$

berechnet.

Nachdem der Translationsvektor Vec_tr ermittelt wurde, wird noch der Zugriff auf die Texturkoordinaten angepasst. Denn ansonsten würde eine Textur eines Abtastvolumens an einer aktuellen Abtastposition pos durch Auslesen der Bilddaten BD an der aktuellen Position pos visualisiert werden. Aufgrund der Translation um den Translationsvektor Vec_tr wird bei dem Schritt 1.VIII eine um den Translationsvektor Vec_tr verschobene Position pos_tr = pos - Vec_tr abgetastet.

Falls die Maskendaten MD an dieser Position das Label der zu verschiebenden Struktur aufweisen, was bei dem Schritt 1.IX geprüft wird, wird nun bei dem Schritt 1.X der Abtastposition pos der Dichtewert der Volumendaten VD(pos_tr) und nicht der Volumendaten VD(pos) zugewiesen, so dass die aktuelle Texturkoordinate bzw. die Textur an der aktuellen Stelle pos im visualisierten Bild verändert wird. Zusätzlich erfolgt bei dem Schritt 1.XI eine Änderung der Textur an der verschobenen Position pos_tr, derart, dass die zu verschiebende Struktur an der ursprünglichen Position transparent ist. Auf diese Weise wird eine doppelte Darstellung der zu verschiebenden Struktur vermieden.

Falls der verschobenen Position pos_tr kein Label der zu verschiebenden Struktur zugeordnet ist, wird bei dem Schritt 1.XII der Abtastposition pos der Dichtewert der ursprünglichen Volumendaten VD(pos) zugewiesen und es erfolgt somit keine Veränderung des visualisierten Bildes an dieser Stelle. D.h. in diesem Fall befindet sich die aktuelle Abtastposition pos außerhalb des Bereichs der zu verschiebenden Struktur.

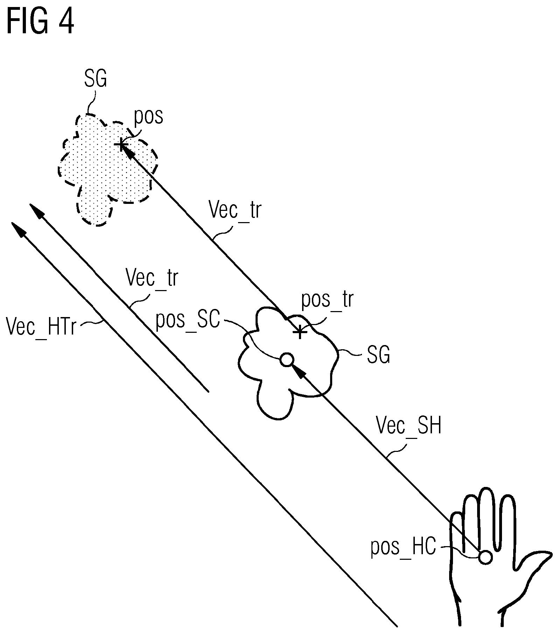

In FIG 4 ist eine schematische Darstellung einer Verschiebung eines Segments von visualisierten Volumendaten in dem in FIG 3 veranschaulichten alternativen Ausführungsbeispiel gezeigt. Das Segment SG soll um den Translationsvektor Vec_tr von der Position pos_SC der Struktur an die aktuelle Position verschoben werden. Einem Bildpunkt an der Stelle pos wird nun nicht die Textur der Position pos in den Volumendaten zugeordnet, sondern die Textur der um den Translationsvektor Vec_tr verschobenen Position pos_tr. Auf diese Weise wird die zu verschiebende Struktur SG von der ursprünglichen Position pos_SC an die aktuelle Stelle verschoben. Der Vektor Vec_SH zwischen Eingabemedium und Struktur SG an der ursprünglichen Position ergibt sich zu

$$\text{Vec\_SH} = \text{pos\_SC} - \text{pos\_HC} \quad (3).$$

Ein weiterer Vektor, der Vektor Vec_HTr zwischen Hand und verschobener Position der Struktur SG ergib sich zu

$$\text{Vec\_HTr} = \text{dist} * (\text{pos\_SC} - \text{pos\_HC}) \quad (4).$$

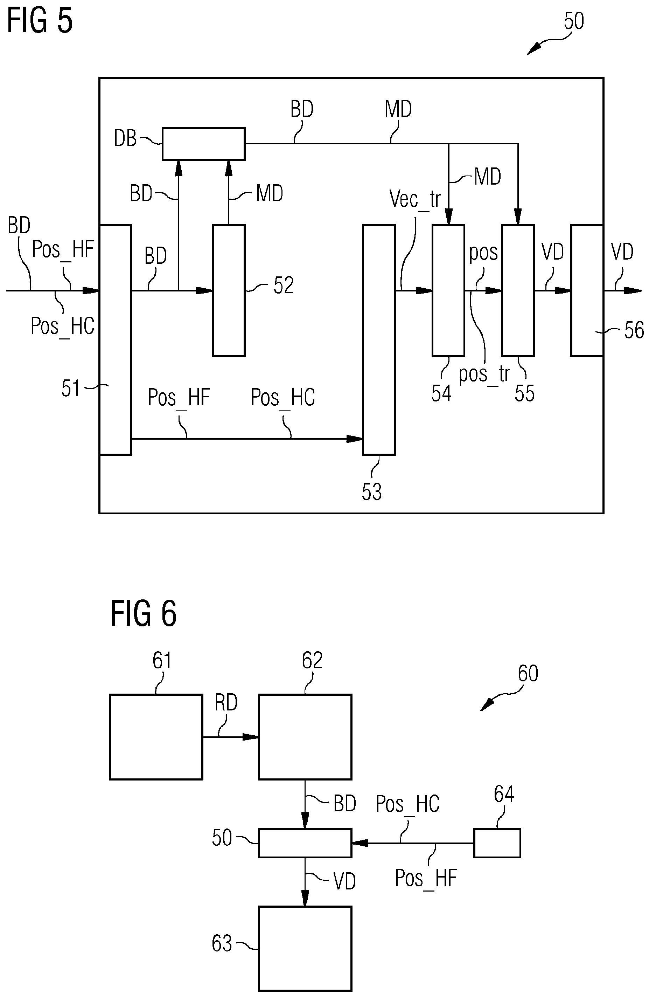

In FIG 5 ist eine schematische Darstellung einer Visualisierungseinrichtung 50 gemäß einem Ausführungsbeispiel der Erfindung gezeigt.

Die Visualisierungseinrichtung 50 weist eine Eingangsschnittstelle 51 als Akquisitionseinheit auf. Die Eingangsschnittstelle 51 empfängt Bilddaten BD eines zu untersuchenden Bereichs eines Patienten von einer medizintechnischen Bildgebungseinrichtung (nicht gezeigt). Die Bilddaten BD werden als Volumendaten VD zur Visualisierung des zu untersuchenden Bereichs benötigt. Weiterhin empfängt die Eingangsschnittstelle 51 auch Daten bezüglich der aktuellen Position pos_HC des Eingabemediums und der Ausgangsposition pos_HF des Eingabemediums. Die Bilddaten BD werden an eine Maskenerzeugungseinheit 52 übermittelt, welche die Bilddaten BD bezüglich einzelner Organe oder anderer Körperstrukturen segmentiert und eine Maske erzeugt, welche Maskendaten MD für alle segmentierten Strukturen umfasst. Die Maskendaten MD weisen segmentierten Bereichen im Bildvolumen bestimmte Klassen bzw. Gewebearten zu. Dabei werden einzelnen Voxeln sogenannte Label zugewiesen, die ein Voxel einer Art von Segment eindeutig zuordnen. Auf diese Weise werden zwei Datensätze erhalten, ein Volumendatensatz mit den Grauwerten der Bilddaten BD sowie entsprechenden Texturdaten und ein Maskendatensatz MD mit den Maskendaten, welche jedes Voxel einem bestimmten Segment bzw. Körperteil zuordnen.

Die beiden Datensätze BD, MD werden in einem Datenspeicher DB zwischengespeichert, der ebenfalls Teil der Visualisierungseinrichtung 50 ist. Die Visualisierungseinrichtung 50 umfasst zudem auch eine Vektorerzeugungseinheit 53, welche auf Basis der von der Eingangsschnittstelle 51 empfangenen Daten bezüglich der aktuellen Position pos_HC des Eingabemediums und der Ausgangsposition pos_HF des Eingabemediums einen Translationsvektor Vec_Tr nach Gleichung (1) oder Gleichung (2) berechnet. Die Daten des Translationsvektors Vec_Tr werden an eine Translationseinheit 54 übermittelt, die ebenfalls Teil der Visualisierungseinrichtung 50 ist. Die Translationseinheit 54 ermittelt auf Basis des Translationsvektors Vec_Tr eine Translationsposition pos_tr und empfängt von dem Datenspeicher dieser Translationsposition pos_tr zugeordnete Label MD, um zu ermitteln, ob an der Translationsposition pos_tr ein Volumensegment des Typs des zu verschiebenden Volumensegments SG vorliegt. Der Typ des zu verschiebenden Volumensegments ergibt sich aus dem Label an der Ausgangsposition pos_HF. Ist dies der Fall, so wird die Translationsposition pos_tr an eine Visualisierungseinheit 55, welche ebenfalls Teil der Visualisierungseinrichtung 50 ist, als Abtastposition übermittelt. Die Visualisierungseinheit 55 ist ebenfalls Teil der Visualisierungseinrichtung 50. Falls an der Translationsposition pos_tr kein Volumensegment des Typs des zu verschiebenden Volumensegments SG vorliegt, so wird von der Translationseinheit 54 die ursprüngliche Abtastpositon pos weiter an die Visualisierungseinheit 55 weitergeleitet und das Raycasting erfolgt an der ursprünglichen Abtastposition pos. Die Visualisierungseinheit 55 bezieht außerdem die in dem Datenspeicher DB abgespeicherten Bilddaten BD und Maskendaten MD. Auf Basis der empfangenen Daten BD, MD sowie der von der Translationseinheit empfangenen Abtastpositon pos, pos_tr erzeugt die Visualisierungseinheit 55 mit Hilfe eines Strahlabtastverfahrens eine perspektivische bildliche Darstellung des zu untersuchenden Bereichs. Die Information über den Translationsvektor Vec_tr bzw. die Translationsposition pos_tr wird von der Visualisierungseinheit 55 dazu genutzt, bei dem Strahlabtastverfahren in dem Bereich des zu verschiebenden Volumensegments SG derart auf die Bilddaten BD bzw. deren zugeordneten Texturen zuzugreifen, dass es zu einer gewünschten Verschiebung der von dem Volumensegment SG umfassten Struktur kommt. Die visualisierten Volumendaten VD werden an eine Ausgangsschnittstelle 56 übermittelt, welche diese Daten VD an eine Bilddarstellungseinheit (nicht gezeigt) weiterleitet.

In FIG 6 ist eine schematische Darstellung eines medizintechnischen Bildgebungssystems 60 gemäß einem Ausführungsbeispiel der Erfindung veranschaulicht. Das medizintechnische Bildgebungssystem 60 weist eine Scaneinheit 61 auf, welche der Akquisition von Rohdaten RD von einem Untersuchungsbereich eines Patienten dient. Die Scaneinheit kann zum Beispiel eine auf dem Prinzip der Computertomographie basierende Scaneinheit umfassen. Die von dieser Scaneinheit 61 akquirierten Rohdaten RD werden an eine Steuereinheit 62 übermittelt, welche sowohl für eine Ansteuerung der Scaneinheit 61 als auch für eine Auswertung der Rohdaten RD und eine Rekonstruktion von Bilddaten BD genutzt wird. Die erzeugten Bilddaten BD werden an eine Visualisierungseinrichtung 50 übermittelt, welche den in FIG 5 gezeigten Aufbau aufweist. Die Visualisierungseinrichtung 50 kann auch über eine Eingabeeinheit 64 angesteuert werden, um zum Beispiel Positionsdaten Pos_HC, Pos_HF von zu verschiebenden Segmenten in zu visualisierenden Bilddaten BD an die Visualisierungseinrichtung 50 zu übermitteln. Die von der Visualisierungseinrichtung 50 erzeugten Visualisierungsdaten bzw. visualisierten Volumendaten VD werden an eine Bilddarstellungseinrichtung 63 übermittelt, welche zum Beispiel einen Monitor umfasst, mit dem die visualisierten Daten VD bildlich dargestellt werden können.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1.  Verfahren zur Visualisierung von medizinischen Bilddaten (BD) als Volumendaten (VD), aufweisend die Schritte:

    - Akquirieren der medizinischen Bilddaten (BD) aufweisend mehrere Voxel,
    - Erzeugen einer 3D-Maske (MD) durch Segmentieren der Bilddaten (BD) und Einteilen der segmentierten Bereiche (SG) in vorbestimmte Klassen,
    - Abspeichern der Bilddaten (BD) und der Maskendaten (MD) in zwei getrennten 3D-Texturen-Dateien, wobei die Maskendaten (MD) Label aufweisen, welche jedem Voxel ein bestimmtes Volumensegment (SG) zuordnen, wobei das Volumensegment (SG) einem segmentierten Bereich (SG) entspricht,
- Berechnen eines Translationsvektors (Vec_tr), welcher die Verschiebung eines Volumensegments (SG) zwischen einer Ursprungsposition (pos_HF) und einer Zielposition (pos_HC) beschreibt,
- Erzeugen einer bildlichen Darstellung der Bilddaten (BD) durch Anwenden eines Strahlabtastverfahrens auf die abgespeicherten Bilddaten (BD),
- Durchführen einer Verschiebung eines Volumensegments (SG) in der bildlichen Darstellung um den Translationsvektor (Vec_tr) dadurch, dass
- für eine Abtastposition (pos) eine um den Translationsvektor (Vec_tr) verschobene Position (pos_tr) abgetastet wird und, falls die Maskendaten (MD) an der verschobenen Position (pos_tr) das Label des zu verschiebenden Volumensegments (SG) aufweisen, der Abtastposition (pos) die Bilddaten (BD) an der verschobenen Position (pos_tr) zugewiesen werden,

wobei das Volumensegment (SG) an der Ursprungsposition (pos_HF) transparent gemacht wird.

2. Verfahren nach dem vorstehenden Anspruch, wobei

- ein vorbestimmter Distanzfaktor (dist) zwischen dem zu verschiebenden Volumensegment (SG) und dem übrigen Volumen vorbestimmt wird und
- der Translationsvektor (Vec_tr) sich aus dem Produkt der Differenz aus dem vorbestimmter Distanzfaktor (dist) und dem Wert 1 und einer Differenz aus der Position (pos_SC) des Zentrums des zu verschiebenden Volumensegments (SG) und einer aktuellen Position (pos_HC) eines Eingabemediums ergibt.

3. Verfahren nach einem der vorstehenden Ansprüche, wobei nahe an dem Eingabemedium liegende Volumensegmente (SG) stärker verschoben werden als von dem Eingabemedium entfernter liegende Volumensegmente (SG).

4. Visualisierungseinrichtung (50), zur Visualisierung von medizinischen Bilddaten(BD) als Volumendaten (VD) aufweisend:

- eine Akquisitionseinheit (51) zum Akquirieren der medizinischen Bilddaten (BD) aufweisend mehrere Voxel,
- eine Maskenerzeugungseinheit (52) zum Erzeugen einer 3D-Maske (MD) durch Segmentieren der Bilddaten (BD) und Einteilen der segmentierten Bereiche (SG) in vorbestimmte Klassen,
- eine Speichereinheit (DB) zum Abspeichern der Bilddaten (BD) und der Maskendaten (MD) in zwei getrennten 3D-Textur-Dateien, wobei die Maskendaten (MD) Label aufweisen, welche jedem Voxel ein bestimmtes Volumensegment (SG) zuordnen, wobei das Volumensegment (SG) einem segmentierten Bereich (SG) entspricht,
- eine Vektorerzeugungseinheit (53) zum Berechnen eines Translationsvektors (Vec_tr), welcher die Verschiebung eines Volumensegments (SG) zwischen einer Ursprungsposition (pos_HF) und einer Zielposition (pos_HC) beschreibt,
- eine Translationseinheit (54) zum Verschieben des Volumensegments (SG) um den Translationsvektor (Vec_tr) in der bildlichen Darstellung,
- eine Visualisierungseinheit (55) zum Erzeugen einer bildlichen Darstellung (VD) der Bilddaten (BD) durch Anwendung eines Strahlabtastverfahrens auf die abgespeicherten Bilddaten (BD) wobei für eine Abtastposition (pos) eine um den Translationsvektor (Vec_tr) verschobene Position (pos_tr) abgetastet wird und, falls die Maskendaten (MD) an der verschobenen Position (pos_tr) das Label des zu verschiebenden Volumensegments (SG) aufweisen, der Abtastposition (pos) die Bilddaten (BD) an der verschobenen Position (pos_tr) zugwiesen werden, wobei das Volumensegment (SG) an der Ursprungsposition (pos_HF) transparent gemacht wird.

5. Medizintechnisches Bildgebungssystem (60), aufweisend:

- eine Visualisierungseinrichtung (50) nach Anspruch 4,
- eine Bildaufnahmeeinheit (61, 62) zum Erzeugen von Bilddaten (BD) von einem Untersuchungsbereich eines zu untersuchenden Objekts.

6. Medizintechnisches Bildgebungssystem nach Anspruch 5, wobei die Bildaufnahmeeinheit eine Scaneinheit eines der folgenden Typen aufweist:

- eine CT-Scaneinheit,
- eine MR-Scaneinheit,

- eine Tomosyntheseeinheit.

7. Computerprogramm, welches direkt in eine Speichereinheit eines medizintechnischen Bildgebungssystems (60) ladbar ist, mit Programmabschnitten, um alle Schritte eines Verfahrens nach einem der Verfahrensansprüche auszuführen, wenn das Computerprogramm in dem medizintechnischen Bildgebungssystem (60) ausgeführt wird.

8. Computerlesbares Medium, auf welchem von einer Rechnereinheit ausführbare Programmabschnitte gespeichert sind, um alle Schritte des Verfahrens nach einem der Verfahrensansprüche auszuführen, wenn die Programmabschnitte von der Rechnereinheit ausgeführt werden.

**Claims**

1. Method for the visualisation of medical image data (BD) as volume data (VD), having the steps:

   - acquiring the medical image data (BD) having a number of voxels,
   - producing a 3D mask (MD) by segmenting the image data (BD) and classifying the segmented regions (SG) into predefined classes,
   - storing the image data (BD) and the mask data (MD) in two separate 3D texture files, wherein the mask data (MD) has labels which assign a specific segmented volume element (SG) to each voxel, wherein the segmented volume element (SG) corresponds to a segmented region (SG),
   - calculating a translation vector (Vec_tr) which describes the displacement of a segmented volume element (SG) between an original position (pos_HF) and a destination position (pos_HC),
   - producing a pictorial display of the image data (BD) by applying a raycasting method to the stored image data (BD),
   - performing a displacement by the translation vector (Vec_tr) of a segmented volume element (SG) in the pictorial display so that
   - for a scanning position (pos) a position (pos_tr) displaced by the translation vector (Vec_tr) is scanned and, if the mask data (MD) at the displaced position (pos_tr) has the label of the segmented volume element (SG) to be displaced, the image data (BD) at the displaced position (pos_tr) is assigned to the scanning position (pos),

   wherein the segmented volume element (SG) is made transparent at the original position (pos_HF).

2. Method according to the preceding claim, wherein

   - a predefined distance factor (dist) is predefined between the segmented volume element (SG) to be displaced and the remaining volume, and
   - the translation vector (Vec_tr) is derived from the product of the difference between the predefined distance factor (dist) and the value 1, and a difference between the position (pos_SC) of the centre of the segmented volume element to be displaced (SG) and a current position (pos_HC) of an input medium.

3. Method according to one of the preceding claims, wherein segmented volume elements (SG) lying close to the input medium are displaced by a greater extent than segmented volume elements (SG) lying further away from the input medium.

4. Visualisation entity (50) for visualising medical image data (BD) as volume data (VD), having:

   - an acquisition unit (51) for acquiring the medical image data (BD) having a number of voxels,
   - a mask production unit (52) for producing a 3D mask (MD) by segmenting the image data (BD) and classifying the segmented regions (SG) into predefined classes,
   - a storage unit (DB) for storing the image data (BD) and the mask data (MD) in two separate 3D texture files, wherein the mask data (MD) has labels which assign a specific segmented volume element (SG) to each voxel, wherein the segmented volume element (SG) corresponds to a segmented region (SG),
   - a vector production unit (53) for calculating a translation vector (Vec_tr) which describes the displacement of a segmented volume element (SG) between an original position (pos_HF) and a destination position (pos_HC),
   - a translation unit (54) for the displacement by the translation vector (Vec_tr) of the segmented volume element (SG) in the pictorial display,
   - a visualisation unit (55) for producing a pictorial display (VD) of the image data (BD) by applying a raycasting method to the stored image data (BD) wherein for a scanning position (pos) a position (pos_tr) displaced by the translation vector (Vec_tr) is scanned, and if the mask data (MD) at the displaced position (pos_tr) has the label of the segment volume element (SG) to be displaced, the image data (BD) at the displaced position (pos_tr) is assigned to the scanning position (pos), wherein the segmented volume element (SG) is made transparent at the original position (pos_HF).

5. Medical imaging system (60), having:

   - a visualisation entity (50) according to claim 4,
   - an image recording unit (61, 62) for producing image data (BD) from an examination region of an object to be examined.

6. Medical imaging system according to claim 5, wherein the image recording unit has a scanning unit of one of the following types:

   - a CT scanning unit,
   - an MR scanning unit,
   - a tomosynthesis unit.

7. Computer program which can be loaded directly into a storage unit of a medical imaging system (60), with program sections for executing all steps of a method according to one of the method claims when the computer program is executed in the medical imaging system (60).

8. Computer-readable medium, on which are stored program sections that can be executed by a computer unit, in order to execute all steps of the method according to one of the method claims when the program sections are executed by the computer unit.

**Revendications**

1. Procédé de visualisation de données (BD) d'image médicales sous la forme de données (VD) de volume, comportant les stades :

   - acquisition des données (BD) d'image médicales comportant plusieurs voxels,
   - production d'un masque (MD) en 3D par segmentation des données (BD) d'image et subdivision des parties (SG) segmentées en des classes déterminées à l'avance,
   - mise en mémoire des données (BD) d'image et des données (MD) de masque dans deux fichiers distincts de textures en 3D, dans lequel les données (MD) de masque ont des étiquettes, qui affectent à chaque voxel un segment (SG) de volume déterminé, dans lequel le segment (SG) de volume correspond à une partie (SG) segmentée,
   - calcul d'un vecteur (Vec_tr) de translation, qui décrit le déplacement d'un segment (SG) de volume entre une position (pos_HF) d'origine et une position (pos_HC) cible,
   - production d'une représentation imagée des données (BD) d'image par application d'un procédé de balayage de rayon aux données (BD) d'image mises en mémoire,
   - exécution d'un déplacement d'un segment (SG) de volume dans la représentation imagée du vecteur (Vec_tr) de translation, **caractérisé en ce que**
   - pour une position (pos) de balayage, une position (pos_tr) décalée du vecteur (Vec_tr) de translation est balayée et, si les données (MD) de masque en la position (pos_tr) décalée ont l'étiquette du segment (SG) de volume déplacé, les données (BD) d'image à la position (pos_tr) déplacée sont affectées à la position (pos) de balayage,

   dans lequel le segment (SG) de volume est rendu transparent à la position (pos_HF) d'origine.

2. Procédé suivant la revendication précédente, dans lequel

   - un facteur (dist) de distance déterminé à l'avance est déterminé à l'avance entre le segment (SG) de volume déplacé et le reste du volume et
   - le vecteur (Vec_tr) de translation provient du produit de la différence entre le facteur (dist) de distance déterminé à l'avance et la valeur 1 par une différence entre la position (pos_SC) du centre du segment (SG) de volume déplacé et une position (pos_HC) en cours d'un milieu d'entrée.

3. Procédé suivant l'une des revendications précédentes, dans lequel des segments (SG) de volume proches du milieu d'entrée sont déplacés davantage que des segments (SG) de volume loin du milieu d'entrée.

4. Dispositif (50) de visualisation, pour la visualisation de données (BD) d'image médicales sous la forme de données (VD) de volume comportant :

- une unité (51) d'acquisition pour l'acquisition des données (BD) d'image médicales comportant plusieurs voxels,
- une unité (52) de production de masse pour la production d'un masque (MD) en 3D par segmentation des données (BD) d'image et subdivision des parties (SG) segmentées en des classes déterminées à l'avance,
- une unité (DB) de mémoire pour la mise en mémoire des données (BD) d'image et des données (MD) de masque dans deux fichiers distincts de texture en 3D, dans lequel les données (MD) de masque ont des étiquettes, qui associent à chaque voxel un segment (SG) de volume déterminé, dans lequel le segment (SG) de volume correspond à une partie (SG) segmentée,
- une unité (53) de production de vecteurs pour le calcul d'un vecteur (Vec_tr) de translation, qui décrit le déplacement d'un segment (SG) de volume entre une position (pos_HF) d'origine et une position (pos_HC) cible,
- une unité (54) de translation pour le déplacement du segment (SG) de volume du vecteur (Vec_tr) de translation dans la représentation imagée,
- une unité (55) de visualisation pour la production d'une représentation (VD) imagée des données (BD) d'image par application d'un procédé de balayage par rayon aux données (BD) d'image mises en mémoire, dans lequel pour une position (pos) de balayage, une position (pos_tr) déplacée du vecteur (Vec_tr) de translation est balayée et, si les données (MD) d'image en la position (pos_tr) déplacée ont l'étiquette du segment (SG) de volume déplacé, les données (BD) d'image en la position (pos_tr) déplacée sont affectées à la position (pos) de balayage, dans lequel le segment (SG) de volume est rendu transparent en la position (pos_HF) d'origine.

5. Système (60) d'imagerie de la technique médicale, comportant :

- un dispositif (50) de visualisation suivant la revendication 4,
- une unité (61, 62) de prise d'image pour la production de données (BD) d'image d'une partie à examiner d'un objet à examiner.

6. Système d'imagerie de la technique médicale suivant la revendication 5, dans lequel l'unité de prise d'image a une unité de balayage de l'un des types suivants :

- une unité de balayage de tomodensitométrie,
- une unité de balayage RM,
- une unité de tomosynthèse.

7. Programme d'ordinateur, qui peut être chargé directement dans une unité de mémoire d'un système (60) d'imagerie de la technique médicale, comportant des parties de programme pour effectuer tous les stades d'un procédé suivant l'une des revendications précédentes, lorsque le programme d'ordinateur est exécuté dans le système (60) d'imagerie de la technique médicale.

8. Support déchiffrable par ordinateur, suivant lequel sont mises en mémoire des parties de programme pouvant être exécutées par une unité informatique, afin d'exécuter tous les stades du procédé suivant l'une des revendications de procédé, lorsque les parties de programme sont exécutées par l'unité informatique.

# FIG 1

100

1.I

BD

1.II

BD    MD

1.III  MD → DB, BD → DB

1.IV

VD

1.VI  Vec_tr=pos_HC - pos_HF

VD

1.VII  VD (pos - Vec_tr)

VD

1.VIII
Label (VD)=
Label (pos-Vec_tr)?  n

j

1.IX  VD(pos_tr) → pos

1.X  VD(pos_tr)=0

1.V

Pos_HC

Pos_HF

VD(pos)  1.XI

## FIG 2

SG

Pos_HC

pos

Vec_tr

Vec_tr

SG

Pos_HF

pos_Tr

# FIG 3

300

```
3.I ─┐ ┌─────────────┐                    ┌─────────┐
     └─│             │                    │         │─ 3.VI
        └──────┬──────┘                    └────┬────┘
               │ ─ BD                           │
               ▼                                │
3.II ─┐ ┌─────────────┐                         │
     └─│             │                          │
        └──────┬──────┘                         │ ─ pos_SC
        BD ─   │ ─ MD                           │
               ▼                                │
3.III ─┐ ┌─────────────┐                        │
      └─│ pos_SC(MD)   │                         │ ─ pos_HC
         └──────┬──────┘                         │
                │ ─ BD                           │
                ▼                                │
3.IV ─┐ ┌────────────────────┐                   │
     └─│ MD → DB, BD → DB     │                   │
        └──────┬─────────────┘                    │
               │                                  │
               ▼                                  │
3.V ─┐ ┌─────────────┐                            │
    └─│             │                             │
       └──────┬──────┘                            │
        VD ─  │                                   │
              ▼                                   │
3.VII ─┐ ┌────────────────────┐                   │
      └─│ Vec_tr=(d-1)*        │◄─────────────────┘
         │ (pos_SC - pos_HC)  │
         └──────┬─────────────┘
      Vec_tr ─  │ ─ VD
                ▼
3.VIII ─┐ ┌────────────────────┐
       └─│ VD (pos - Vec_tr)    │
          └──────┬─────────────┘
           VD ─  │
                 ▼
              ╱─────────╲
3.IX ─┐      ╱ Label (VD)= ╲           n
     └─────▶◄  Label (pos-Vec_tr)? ▶──────────┐
              ╲           ╱                    │
               ╲─────────╱                     │
                   │ j                         │
                   ▼                           │
3.X ─┐ ┌────────────────────┐                  │
    └─│ VD(pos_tr) → pos     │                  │
       └──────┬─────────────┘                   │
              │                                 │
              ▼                                 ▼
3.XI ─┐ ┌────────────────────┐       ┌─────────────┐
     └─│ VD(pos_tr)=0         │       │ VD(pos)     │─ 3.XII
        └────────────────────┘        └─────────────┘
```

FIG 4

# FIG 5

# FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5825365 A

**In der Beschreibung aufgeführte Nicht-Patentliteratur**

- Exploded Views for Volume Data. **STEFAN BRUCKNER et al.** IEEE TRANSACTIONS ON VISUALIZATION AND COMPUTER GRAPHICS. IEEE SERVICE CENTER, 01 September 2006, vol. 12, 1077-1084
- **KANG D S et al.** An interactive exploded view generation using block-based re-rendering. *MEDICAL IMAGING 2011: VISUALIZATION, IMAGE-GUIDED PROCEDURES, AND MODELING; SPIE, 1000 20TH ST. BELLINGHAM WA 98225-6705 USA*, 03 March 2011, vol. 7964 (1), 1-7
- **SUBRAMANIAN N et al.** Volume rendering segmented data using 3D textures: a practical approach for intra-operative visualization. *PROCEEDINGS OF THE SPIE - THE INTERNATIONAL SOCIETY FOR OPTICAL ENINEERING USA*, 2006, vol. 6141, 614120-1
- **HENRY SONNET et al.** Integrating expanding annotations with a 3D explosion probe. *AVI'04 INTERNATIONAL CONFERENCE ON ADVANCED VISUAL INTERFACES; GALLIPOLI, ITALY; MAY 25-28, 2004, ASSOCIATION FOR COMPUTING MACHINERY, NEW YORK, NY, USA*, 25 May 2004, 63-70
- Automated generation of interactive 3D exploded view diagrams. **WILMOT LI et al.** ACM TRANSACTIONS ON GRAPHCS. ACM, 01 August 2008, vol. 27, 1-7